# EUROPEAN PATENT SPECIFICATION

(11) **EP 1 593 392 B1**
(45) Date of publication and mention of the grant of the patent: **28.09.2011**
(21) Application number: 04023168.0
(22) Date of filing: 29.09.2004
(51) Int. Cl.: A61K 39/205, A61K 39/42, C07K 16/10

(54) **Rabies vaccine**
Tollwut-Impfstoff
Vaccin antirabique

(30) Priority: 07.05.2004 US 569172 P
(43) Date of publication of application: 09.11.2005
(73) Proprietor: Novartis Vaccines and Diagnostics GmbH, 35041 Marburg (DE)
(72) Inventor: Banzhoff, Angelika, Dr., 35039 Marburg (DE); Malerczyk, Claudius, Dr., 35039 Marburg (DE)
(74) Representative: UEXKÜLL & STOLBERG

(56) References cited:
- WO-A-02/078732
- BISWAS SUBHABRATA ET AL: "Preexposure efficacy of a novel combination DNA and inactivated rabies virus vaccine" HUMAN GENE THERAPY, vol. 12, no. 15, 10 October 2001 (2001-10-10), pages 1917-1922, XP002306647 ISSN: 1043-0342
- LODMELL D L ET AL: "Post-exposure DNA vaccination protects mice against rabies virus" VACCINE, BUTTERWORTH SCIENTIFIC. GUILDFORD, GB, vol. 19, no. 17-19, 21 March 2001 (2001-03-21), pages 2468-2473, XP004231067 ISSN: 0264-410X
- SHIMAZAKI Y ET AL: "Immune response to Japanese rabies vaccine in domestic dogs." JOURNAL OF VETERINARY MEDICINE SERIES B, vol. 50, no. 2, March 2003 (2003-03), pages 95-98, XP002306648 ISSN: 0931-1793
- ROOIJAKKERS E J M ET AL: "Potency of veterinary rabies vaccines in the Netherlands: A case for continued vigilance" VETERINARY QUARTERLY, vol. 18, no. 4, 1996, pages 146-150, XP008038725 ISSN: 0165-2176
- LODMELL D L ET AL: "Rabies cell culture vaccines reconstituted and stored at 4<o>C for 1 year prior to use protect mice against rabies virus" VACCINE, BUTTERWORTH SCIENTIFIC. GUILDFORD, GB, vol. 22, no. 25-26, 3 September 2004 (2004-09-03), pages 3237-3239, XP004526895 ISSN: 0264-410X
- ANONYMOUS: INTERNET ARTICLE, [Online] November 1996 (1996-11), XP002306650 Retrieved from the Internet: URL:http://www.vetpharm.unizh.ch/TAK/00000 000/00001404.VAK> [retrieved on 2004-11]
- BARTH R ET AL: "NIH TEST A PROBLEMATIC METHOD FOR TESTING POTENCY OF INACTIVATED RABIES VACCINE" VACCINE, vol. 6, no. 4, 1988, pages 369-377, XP002306651 ISSN: 0264-410X
- WARRELL M J ET AL: "Rabies and other lyssavirus diseases." LANCET. 20 MAR 2004, vol. 363, no. 9413, 20 March 2004 (2004-03-20), pages 959-969, XP004497225 ISSN: 1474-547X
- ANONYMOUS: "Intradermal application of rabies vaccines" REPORT OF A WHO CONSULTATION, [Online] 2000, XP002306653 Retrieved from the Internet: URL:http://www.who.int/emc-documents/rabie s/docs/whocdscsraph2005.pdf> [retrieved on 2004-11]
- MADHUSUDANA S N ET AL: "Simulated post-exposure rabies vaccination with purified chick embryo cell vaccine using a modified Thai Red Cross regimen." INTERNATIONAL JOURNAL OF INFECTIOUS DISEASES : IJID : OFFICIAL PUBLICATION OF THE INTERNATIONAL SOCIETY FOR INFECTIOUS DISEASES. MAY 2004, vol. 8, no. 3, May 2004 (2004-05), pages 175-179, XP002306654 ISSN: 1201-9712 -& MADHUSUDANA S N: "Simulated post-exposure rabies vaccination: authors' response to Wilde et al" INTERNATIONAL JOURNAL OF INFECTIOUS DISEASES, INTERNATIONAL SOCIETY FOR INFECTIOUS DISEASES, HAMILTON, CA, vol. 8, no. 6, November 2004 (2004-11), page 376, XP004604576 ISSN: 1201-9712 -& WILDE H ET AL: "Simulated post-exposure rabies vaccination: comments on article by Madhusudana et al" INTERNATIONAL JOURNAL OF INFECTIOUS DISEASES, INTERNATIONAL SOCIETY FOR INFECTIOUS DISEASES, HAMILTON, CA, vol. 8, no. 6, November 2004 (2004-11)
- BRIGGS D J ET AL: "Antibody response of patients after postexposure rabies vaccination with small intradermal doses of purified chick embryo cell vaccine or purified Vero cell rabies vaccine" BULLETIN OF THE WORLD HEALTH ORGANIZATION, vol. 78, no. 5, 2000, pages 693-698, XP008038719 ISSN: 0042-9686
- "IMOVAX Rabies"[Online] April 2000 (2000-04), XP002306655 Retrieved from the Internet: URL:http://www.aventispasteur.ca/avp_conte nt/docs/ca_products/IMOVAX_E.pdf> [retrieved on 2004-11]
- JENKINS ET AL.: "Compendium of Animal Rabies Prevention and Control, 2003" INTERNET ARTICLE, [Online] 2004, XP002306656 Retrieved from the Internet: URL:http://www.avma.org/pubhlth/rabcont.as p> [retrieved on 2004-11]

## Description

The invention provides for an immunogenic rabies vaccine comprising a reduced vaccine dose and methods of post-exposure immunization with a reduced dose. The concentration of rabies vaccine antigen per dose is less than 2.5 IU/mL. For example, the concentration of rabies vaccine antigen may be less than half, less than one fourth, or less than one eighth of 2.5 IU/mL. Preferably, the concentration of rabies vaccine antigen is between 2.4 IU/mL and 0.25 IU/mL, more preferably between 2.0 IU/mL and 1.0 IU/mL and even more preferably between 1.75 IU/mL and 1.25 IU/mL, wherein 1.5 IU/mL is most preferred. This means, the concentration of the rabies virus vaccine antigen in the composition can be 2.2 IU/mL, 1.8 IU/mL, 1.6 IU/mL, 1.4 IU/mL, 1.2 IU/mL, 0.8 IU/mL, 0.6 IU/mL, 0.4 IU/mL, 0.3 IU/mL, or 0.25 IU/mL. The composition is a single-dose unit having a volume of 0.1 mL, the vaccines of the invention are administered intradermally as part of a Thai Red Cross (TRD) or the modified Thai Red Cross regimen.

### BRIEF DESCRIPTION OF THE FIGURES

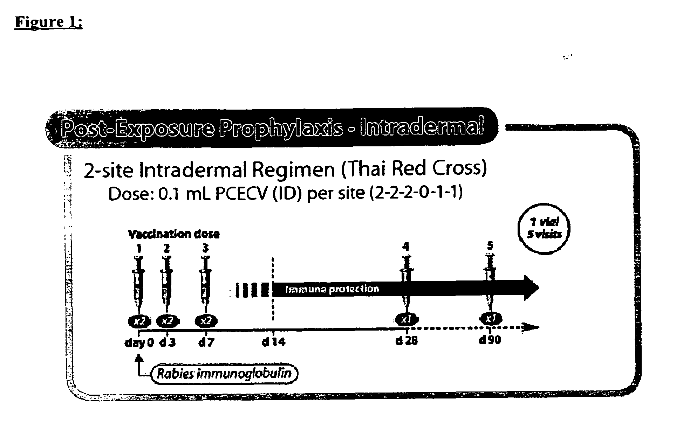

Figure 1 shows a schematic depiction of the post-exposure 2-site intradermal regimen (also known as Thai Red Cross Regimen). Doses of 0.1 mL PCECV are administered intradermally at two different sites of the patient's body. Optionally, rabies immunoglobulin can be given together with the first vaccine dose.

### BACKGROUND OF THE INVENTION

Despite the availability of modern cell culture rabies vaccines, human rabies deaths continue to occur in developing countries where canine rabies is endemic and the cost of post-exposure prophylaxis is unaffordable for the majority of the population. Current WHO recommendations recommend a concentration of rabies virus vaccine derived from cell culture vaccines of at least 2.5 IU/mL. To overcome the financial burden, the WHO has recommended two intradermal (ID) post-exposure rabies treatment regimens that have been successfully implemented in several countries (cf. WHO recommendations on Rabies Post-Exposure Treatment and the Correct Technique of Intradermal Immunization against Rabies [WHO/ECM/ZOO/96.6], WHO Doc 1996.). The more economic of these regimens is the Thai Red Cross ID 2-site (TRC) regimen. The TRC regimen, using 0.1 mL per ID dose, reduces the cost of vaccine by up to 80%. Purified chick embryo cell vaccine (PCECV, such as Rabipur®) has been proven to be highly immunogenic, well tolerated and efficacious in this regimen. Despite the reduced cost for these ID regimens, a need for less expensive rabies immunization, particularly in developing countries, persists.

### BRIEF SUMMARY OF THE INVENTION

Applicants have surprisingly discovered that sufficient neutralizing antibody titers can be achieved by immunization with a substantially reduced rabies virus vaccine antigen concentration. In particular, rabies virus vaccine antigen concentrations of substantially less than the current WHO recommended 2.5 IU/mL can provide neutralizing antibody titers similar to those generated with the standard dose.

The invention provides for a composition comprising a rabies virus vaccine, wherein the concentration of the rabies virus vaccine antigen is less than 2.5 IU/mL. For example, the concentration of the vaccine antigen may be less than half, less than one fourth, or less than one eighth of 2.5 IU/mL. In other words, the concentration of the rabies virus vaccine antigen in the composition is preferably less than 1.25 IU/mL, more preferably less than 0.625 IU/mL, and even more preferably less than 0.3125 IU/mL. Preferably, the concentration of rabies vaccine antigen is between 2.4 IU/mL and 0.25 IU/mL, more preferably between 2.0 IU/mL and 1.0 IU/mL and even more preferably between 1.75 IU/mL and 1.25 IU/mL, wherein 1.5 IU/mL is most preferred. Preferably, the composition is a single-dose unit having a volume of 0.1 mL. This means, the concentration of the rabies virus vaccine antigen in the composition can be 2.2 IU/mL, 1.8 IU/mL, 1.6 IU/mL, 1.4 IU/mL, 1.2 IU/mL, 0.8 IU/mL, 0.6 IU/mL, 0.4 IU/mL, 0.3 IU/mL, or 0.25 IU/mL. Preferably, the rabies virus vaccine is produced in a continuous cell line such as a purified chicken embryo cell line, a purified vero cell line, or a human diploid cell line. Processes and means for the production of rabies antigen which make use of these cell lines are described, for example, in "Vaccines", Plotkin and Orenstein (eds.), 2004, Chapter 37, "Rabies Vaccine", by Plotkin, Rupprecht, Koprowski.

The vaccines of the invention are given therapeutically as part of a complete post exposure regimen and include administration intradermally.

### DETAILED DESCRIPTION OF THE INVENTION

The practice of the present invention will employ, unless otherwise indicated, conventional methods of chemistry, biochemistry, molecular biology, immunology and pharmacology, within the skill of the art. Such techniques are explained fully in the literature. See, e.g., Remington's Pharmaceutical Sciences, Mack Publishing Company, Easton, Pa., 19th Edition (1995); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Handbook of Surface and Colloidal Chemistry (Birdi, K.S. ed., CRC Press, 1997); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); PCR (Introduction to Biotechniques Series), 2nd ed. (Newton & Graham eds., 1997, Springer Verlag); Peters and Dalrymple, Fields Virology (2d ed), Fields et al. (eds.), B.N. Raven Press, New York, NY.

The rabies virus belongs to the family Rhabdoviridae, genus *Lyssavirus* and comprises an enveloped virion with a single, nonsegmented, negative-stranded RNA. The rabies virus is bullet-shaped with trimers of a the rabies glycoprotein (G) projecting to the exterior of the virus. This G protein is thought to be the major antigen responsible for inducing production of neutralizing antibodies and for conferring immunity against lethal infection with rabies virus.

As used herein, the term "International Units" or "IU" of rabies virus vaccine antigen refers to the international potency concentration measurement used by, for example, the WHO and other international health regulatory agencies (see for example, European Pharmacopoeia, 01/2005:0216, Rabies vaccine for human use prepared in cell cultures,. International Units indicate the predicted potency of a vaccine composition and can be measured using standard assays. For example, the rabies vaccine antigen concentration of a sample inactivated rabies virus vaccine can be calculated using a standard assay to measure the potency of the test vaccine in mouse challenge experiments compared to the potency of a publicly available international reference standard. These standard assays include the NIH mouse potency test and the European Pharmacopoeia test (*see* WHO Expert Committee on Biological Standardization. Thirty-seventh report. Geneva, World Health Organization, WHO Technical Report Series, (1987) No. 760 and (1994) No. 840.

For example, groups of at least ten mice, aged 3 - 4 weeks, are inoculated with single, decreasing doses of vaccine (in accordance with the European Pharmacopoeia) or with two doses, 1 week apart (in accordance with the NIH test). A sufficient number of dilutions of vaccine are compared to estimate the dilution at which 50% of the mice are protected against intracerebral challenge 14 days later (50% protective dose = PD₅₀). A WHO international standard vaccine is available to compare the titration curve of the test vaccine to that of the IU standard, i.e., to ensure that the PD₅₀ of the test vaccine is at least equivalent to the IU standard. The following parameters should be met in order for the titration curve calibration to be valid: (i) for both the test vaccine and the international standard, the PD₅₀ should lie between the largest and the smallest doses given to the mice; (*ii*) the titration of the challenge virus suspension should show that 0.03 mL of the suspension contained at least 10 LD₅₀ (the LD₅₀ should be in the range of 12 - 50 for a valid test); (*iii*) the confidence interval (*p* = 0.95) for the test is not less than 25% and not more than 400% of the estimated potency (i.e., statistical analysis shows a significant slope and no significant deviations from linearity or parallelism of the dose/response lines).

Alternatively, the IU of a rabies vaccine dose may be determined using an antibody binding test (ABBT), which measurement is then converted into IU/mL by comparison with the international standard. See Barth, "The modified antibody binding test for in vitro qualification of rabies virus antigen in inactivated rabies vaccines", in Laboratory Techniques in Rabies (Meslin and Kaplan, eds.) WHO, Geneva 1998, p. 392 - 4.

The rabies inactivated vaccine international standard may be obtained, for example, from the International Laboratory for Biological Standards, Statens Seruminstitute, Copenhagen, Denmark.

The vaccines of the invention may be derived from any of the known tissue or cell culture techniques for rabies known in the art, including for example those used with mammalian cells, baculoviruses, bacteria, or yeast. Suitable cell lines may be used to cultivate the rabies virus or to express polynucleotides encoding rabies viral antigens (such as the G protein).

Preferably, the rabies virus is cultivated in cells of mammalian origin. Such cell lines include but are not limited to: human or non-human primate cells (e.g. MRC-5 (ATCC CCL-171), WI-38 (ATCC CCL-75), human embryonic kidney cells (293 cells, typically transformed by sheared adenovirus type 5 DNA), VERO cells from monkey kidneys), horse, cow (e.g. MDBK cells), sheep, dog (e.g. MDCK cells from dog kidneys, ATCC CCL34 MDCK (NBL2) or MDCK 33016, deposit number DSM ACC 2219 as described in WO97/37001), cat, and rodent (e.g. hamster cells such as BHK21-F, HKCC cells, or Chinese hamster ovary cells (CHO cells)), and may be obtained from a wide variety of developmental stages, including for example, adult, neonatal, fetal, and embryo. In certain embodiments the cells are immortalized (e.g. PERC.6 cells, as described in WO01/38362 and WO02/40665, and as deposited under ECACC deposit number 96022940).

In preferred embodiments, mammalian cells are utilized, and may be selected from and/or derived from one or more of the following non-limiting cell types: fibroblast cells (e.g. dermal, lung), endothelial cells (e.g. aortic, coronary, pulmonary, vascular, dermal microvascular, umbilical), hepatocytes, keratinocytes, immune cells (e.g. T cell, B cell, macrophage, NK, dendritic), mammary cells (e.g. epithelial), smooth muscle cells (e.g. vascular, aortic, coronary, arterial, uterine, bronchial, cervical, retinal pericytes), melanocytes, neural cells (e.g. astrocytes), prostate cells (e.g. epithelial, smooth muscle), renal cells (e.g. epithelial, mesangial, proximal tubule), skeletal cells (e.g. chondrocyte, osteoclast, osteoblast), muscle cells (e.g. myoblast, skeletal, smooth, bronchial), liver cells, retinoblasts, and stromal cells. WO97/37000 and WO97/37001 describe production of animal cells and cell lines that are capable of growth in suspension and in serum free media and are useful in the production and replication of viruses.

Preferred rabies virus vaccine cultivation substrates include purified chick embryo cells, purified vero cells, human diploid cells, rhesus diploid cells, hamster kidney cells, and duck embryo cells. Rabies virus vaccines for use in the invention include purified chicken embryo cell vaccines (PCECV) such as Rabipur® (obtainable from Chiron-Behring, Marburg, Germany) and RabAvert® obtainable from Chiron Corp., Emeryville, USA; purified vero cell vaccine (PVRV) such as Verorab™ (obtainable from Aventis Pasteur) and Immovax-Rabies vero™ (obtainable from Aventis Pasteur); chromatographically purified Vero cell culture rabies vaccine (CPRV); human diploid cell vaccine (HDCV) such as Rabivac® (obtainable from from Chiron-Behring, Marburg, Germany); rhesus diploid cell culture vaccine (RVA) such as fetal rhesus monkey lung fibroblast cultures form BioPort; primary Hamster Kidney Cell Culture Vaccine (PHKCV); and purified duck embryo vaccine (PDEV) such as Lyssavax N™. Preferred rabies virus vaccines for use in the invention include PCECV, PVRV, and HDCV. Processes and means for the production of rabies antigen which make use of these cell lines are described, for example, in "Vaccines", Plotkin and Orenstein (eds.), 2004, Chapter 37, "Rabies Vaccine", by Plotkin, Rupprecht, Koprowski.

In one embodiment, a PCECV inactivated rabies vaccine is prepared by cultivation of the Flury low egg passage (LEP) rabies strain in primary chick embryo fibroblasts (see Example 1). The virus may be cultivated in a synthetic cell culture medium, optionally also including human albumin, polygeline and antibiotics. The virus is then inactivated by approximately 0.025% β-propiolactone, concentrated and purified by zonal centrifugation in a sucrose density gradient, and lyophlizied. Further discussion of commonly used cell cultures for rabies virus cultivation can be found in Vaccines, Plotkin and Orenstein eds., 4th Ed. 2004, Chapter 37 (pp 1011 - 1038, see in particular pp 1018 - 1022).

Alternatively, the vaccines of the invention may be cultivated in bacterial, insect, or plant cell culture systems. Insect cell expression systems, such as baculovirus systems, are known to those of skill in the art and described in, e.g., Summers and Smith, *Texas Agricultural Experiment Station Bulletin* No. 1555 (1987). Materials and methods for baculovirus/insert cell expression systems are commercially available in kit form from, *inter alia,* Invitrogen, San Diego CA. Insect cells for use with baculovirus expression vectors include, *inter alia, Aedes aegypti, Autographa californica, Bombyx mori, Drosophila melanogaster, Spodoptera frugiperda,* and *Trichoplusia ni.*

Similarly, bacterial and mammalian cell expression systems are also known in the art and described in, *e.g., Yeast Genetic Engineering* (Barr *et al.,* eds., 1989) Butterworths, London. Bacterial hosts such as *E. coli, Bacillus subtilis,* and *Streptococcus spp.,* could be used to express constructs encoding rabies viral antigens. Yeast hosts useful in the present invention include, *inter alia, Saccharomyces cerevisiae, Candida albicans, Candida maltosa, Hansenual polymorpha, Kluyveromyces fragilis, Kluyveromyces lactis, Pichia guillerimondii, Pichia pastoris, Schizosaccharomyces pombe* and *Yarrowia lipolytica*.

Nucleic acid molecules comprising nucleotide sequences of the viral antigens or antibodies of the invention can be stably integrated into a host cell genome or maintained on a stable episomal element in a suitable host cell using various gene delivery techniques well known in the art. See., *e.g.*, US Patent No. 5,399,346.

Depending on the expression system and host selected, the molecules are produced by growing host cells transformed by an expression vector under conditions whereby the protein is expressed. The expressed protein is then isolated from the host cells and purified. If the expression system secretes the protein into growth media, the product can be purified directly from the media. If it is not secreted, it can be isolated from cell lysates. The selection of the appropriate growth conditions and recovery methods are within the skill of the art.

The rabies vaccines of the invention preferably comprise inactivated rabies virus cultivated in mammalian cells. General methods of inactivating or killing viruses to destroy their ability to infect mammalian cells are known in the art. Such methods include both chemical and physical means. Chemical means for inactivating a virus include treatment with an effective amount of one or more of the following agents: detergents, formaldehyde, β-propiolactone, or UV light. Additional chemical means for inactivation include treatment with methylene blue, psoralen, carboxyfullerene (C60) or a combination of any thereof. Other methods of viral inactivation are known in the art, such as for example binary ethylamine, acetyl ethyleneimine, or gamma irradiation. The preferred method of inactivation for rabies virus is treatment with β-propiolactone.

The rabies vaccines of the invention may comprise further components such as human serum albumin, gelatin, phenolsulfonphthalein, or antibiotics such as neomycin, chlortetracycline, and amphotericin B.

The vaccines of the invention may be lyophlilized to a powder form for storage or shipment, and reconstituted in sterile water for immunization.

The vaccines of the invention are preferably stored at cool temperatures, preferably 2° to 8° C.

The vaccines of the invention may be prepared using one or more concentration techniques such as zonal centrifugation, density gradient centrifugation, ultrafiltration, and chromatography.

The invention includes use of reduced concentration rabies virus vaccines in post-exposure dosing regimens by immunization via intradermal administration. The intradermal doses generally comprise a total volume of 0.1 mL.

The invention provides for a method of immunizing a subject with a rabies vaccine comprising injecting the subject with one or more doses of rabies vaccine, wherein the concentration of the dose of rabies vaccine antigen is less than 2.5 IU/mL. For example, the concentration of the vaccine antigen may be less than half, less than one fourth, or less than one eighth of 2.5 IU/mL. Preferably, the concentration of rabies vaccine antigen is between 2.4 IU/mL and 0.25 IU/mL, more preferably between 2.0 IU/mL and 1.0 IU/mL and even more preferably between 1.75 IU/mL and 1.25 IU/mL, wherein 1.5 IU/mL is most preferred. This means, the concentration of the rabies virus vaccine antigen in the composition can be 2.2 IU/mL, 1.8 IU/mL, 1.6 IU/mL, 1.4 IU/mL, 1.2 IU/mL, 0.8 IU/mL, 0.6 IU/mL, 0.4 IU/mL, 0.3 IU/mL, or 0.25 IU/mL.

Post-exposure dosing regimens are administered intradermally. Preferred post-exposure rabies vaccines include PCECV, HDCV or RVA vaccines. The invention thus provides for a method of post-exposure immunization against rabies infection comprising immunizing a subject who has been exposed to rabies infection with a rabies vaccine, wherein the concentration of the dose of rabies vaccine antigen is less than 2.5 IU/mL. Preferably, the concentration of rabies vaccine antigen is between 2.4 IU/mL and 0.25 IU/mL, more preferably between 2.0 IU/mL and 1.0 IU/mL and even more preferably between 1.75 IU/mL and 1.25 IU/mL, wherein 1.5 IU/mL is most preferred. This means, the concentration of the rabies virus vaccine antigen in the composition can be 2.2 IU/mL, 1.8 IU/mL, 1.6 IU/mL, 1.4 IU/mL, 1.2 IU/mL, 0.8 IU/mL, 0.6 IU/mL, 0.4 IU/mL, 0.3 IU/mL, or 0.25 IU/mL. The vaccine is administered in 0.1 mL doses.

Intradermal post-exposure dosing regimens for use in the invention include a two site intradermal method known as ("2-2-2-0-1-1"; the numerical abbreviation refers to the numbers of doses administered on days 0,3,7,14,28, and 90; i.e. the number of doses administered on the administration days scheduled in accordance with the standard WHO intramuscular regimen). The "2-2-2-0-1-1" regimen is also known as the Thai Red Cross regimen or TRC-ID. This regimen comprises intradermal administration of 0.1 mL at two sites (such as the upper arm or over each deltoid) at each of days 0, 3 and 7. This regimen further comprises intradermal administration of 0.1 mL at one site (such as the upper arm) on days 28 and 90.

A modified version of the TRC-ID is designated ("2-2-2-0-2"). This regimen comprises intradermal immunization of 0.1 mL at two sites on days 0, 3, 7 and 28 (with the 90 day booster being omitted).

For example, the rabies vaccine according to the invention may be administered after contact with an animal which might be rabid. According to the WHO recommendations for Rabies Postexposure Prophylaxis (WHO Expert Committee on Rabies; World Organisation Tech. Rep. Ser. 824:1-84 (1992)), vaccination should be performed immediately after an animal suspected or confirmed of being rabid (upon contact with a human) caused minor scratches or abrasions without bleeding, single or multiple transdermal bites or scratches, or even after nibbling of uncovered skin. However, post-exposure treatment can be stopped after a 10 days-observation period in which the animal remains healthy. In these cases, the costs of vaccinating can be considerably reduced by administering the vaccines of the invention. For example, only two 0.1 mL doses mL at two sites (such as the upper arm or over each deltoid) on days 0, 3 and 7 are sufficient to produce a protective antibody titer of 0.5 IU/mL or more. Preferably, these doses can have an antigen concentration of about 1.5 IU/mL, 1.0 IU/mL or even 0.5 IU/mL or 0.25 IU/mL. Alternative regimens comprise two 0.1 mL doses on days 0, 5 and 8, or single 0.1 mL doses on days 0, 2, 4, 6 or on days 0, 3, 5, 7.

In the dosing regimens of the invention requiring intradermal administration, the intradermal dose volume is about 0.1 mL. The rabies virus vaccine antigen concentration per dose in intradermal dosing regimens is consequently less than 2.5 IU/mL (i.e., less than 0.25 IU/0.1mL). Preferably the rabies vaccine antigen concentration in an intradermal dosing regimen is less than one half the concentration of 2.5 IU/mL (i.e., less than 0.13 IU/0.1mL), less than one fourth the concentration of 2.5 IU/mL (i.e., less than 0.06 IU/mL), or less than one eighth the concentration of 2.5 IU/mL (i.e., less than 0.03 IU/0.1mL).

The reduced dose rabies vaccines of the invention generate neutralizing antibody titers in the subject suitable for meeting international regulatory standards. Preferably, the vaccines of the invention provide for subject antibody titers greater than 0.5 IU/mL. Measurement of neutralizing antibody titers in rabies vaccine immunized subjects is known in the art and may be accomplished by, for example, the rapid fluorescent focus inhibition test (RFFIT).

Post-exposure immunization regimens should be accompanied by administration of rabies immune globulin (RIG). Preferably, RIG is administered as a single dose of 20 IU per kg of body weight for human RIG or as a single dose of 40 IU per kg of body weight for heterologous (equine) RIG. This single dose of RIG is preferably given at the same time as the first dose of vaccine. If RIG is unavailable at day 0, it may be administered up to day 7.

For subjects given pre-exposure vaccine regimens, a post exposure boost of vaccine given on days 0 and 3 is generally recommended. Such post exposure boosts could be intramuscular or intradermal doses.

Subjects suitable for immunization with the vaccines of the invention are humans.

Also disclosed herein is the use of a rabies virus antigen for the preparation of a medicament for the treatment of rabies virus infection, wherein the concentration of rabies virus antigen is between 2.4 IU/mL and 0.25 IU/mL, preferably between 2.0 IU/mL and 1.0 IU/mL, more preferably between 1.75 IU/mL and 1.25 IU/mL, and most preferably 1.5 IU/mL. Preferably, this treatment is a post-exposure treatment. Further, it is preferred that the medicament is administered via intradermal administration. The medicament preferably is to be administered in at least two doses in a period of ten days after contact with the animal. Preferably, the medicament is to be administered in at least three, four, five, six or seven doses in a period of ten days after after contact with the animal. Usally, the doses have a volume of 0.1 mL.

While the use of adjuvants are not required to obtain sufficient levels of neutralizing antibody titers, the reduced vaccine doses of the invention may be supplemented with one or more adjuvants. Adjuvants for use with the invention include, but are not limited to, one or more of the following set forth below:

### A. Mineral Containing Compositions

Mineral containing compositions suitable for use as adjuvants in the invention include mineral salts, such as aluminum salts and calcium salts. The invention includes mineral salts such as hydroxides (e.g. oxyhydroxides), phosphates (e.g. hydroxyphosphates, orthophosphates), sulfates, etc. (e.g. see chapters 8 & 9 of Vaccine Design... (1995) eds. Powell & Newman. ISBN: 030644867X. Plenum.), or mixtures of different mineral compounds (e.g. a mixture of a phosphate and a hydroxide adjuvant, optionally with an excess of the phosphate), with the compounds taking any suitable form (e.g. gel, crystalline, amorphous, etc.), and with adsorption to the salt(s) being preferred. The mineral containing compositions may also be formulated as a particle of metal salt (WO00/23105).

Aluminum salts may be included in vaccines of the invention such that the dose of Al³⁺ is between 0.2 and 1.0 mg per dose.

### B. Oil-Emulsions

Oil-emulsion compositions suitable for use as adjuvants in the invention include squalene-water emulsions, such as MF59 (5% Squalene, 0.5% Tween 80, and 0.5% Span 85, formulated into submicron particles using a microfluidizer). See WO90/14837. See also, Podda, "The adjuvanted influenza vaccines with novel adjuvants: experience with the MF59-adjuvanted vaccine", Vaccine (2001) 19: 2673-2680. MF59 is used as the adjuvant in the FLUAD™ influenza virus trivalent subunit vaccine.

Particularly preferred adjuvants for use in the compositions are submicron oil-in-water emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80™ (polyoxyelthylenesorbitan monooleate), and/or 0.25-1.0% Span 85™ (sorbitan trioleate), and, optionally, N-acetylmuramyl-L-alanyl-D-isogluatminyl-L-alanine-2-(1'-2'-dipalmitoyl-sn-glycero-3-huydroxyphosphophoryloxy)-ethylamine (MTP-PE), for example, the submicron oil-in-water emulsion known as "MF59" (International Publication No. WO90/14837; US Patent Nos. 6,299,884 and 6,451,325, incorporated herein by reference in their entireties; and Ott et al., "MF59 -- Design and Evaluation of a Safe and Potent Adjuvant for Human Vaccines" in Vaccine Design: The Subunit and Adjuvant Approach (Powell, M.F. and Newman, M.J. eds.) Plenum Press, New York, 1995, pp. 277-296). MF59 contains 4-5% w/v Squalene (e.g. 4.3%), 0.25-0.5% w/v Tween 80™, and 0.5% w/v Span 85™ and optionally contains various amounts of MTP-PE, formulated into submicron particles using a microfluidizer such as Model 110Y microfluidizer (Microfluidics, Newton, MA). For example, MTP-PE may be present in an amount of about 0-500 µg/dose, more preferably 0-250 µg/dose and most preferably, 0-100 µg/dose. As used herein, the term "MF59-0" refers to the above submicron oil-in-water emulsion lacking MTP-PE, while the term MF59-MTP denotes a formulation that contains MTP-PE. For instance, "MF59-100" contains 100 µg MTP-PE per dose, and so on. MF69, another submicron oil-in-water emulsion for use herein, contains 4.3% w/v squalene, 0.25% w/v Tween 80™, and 0.75% w/v Span 85™ and optionally MTP-PE. Yet another submicron oil-in-water emulsion is MF75, also known as SAF, containing 10% squalene, 0.4% Tween 80™, 5% pluronic-blocked polymer L121, and thr-MDP, also microfluidized into a submicron emulsion. MF75-MTP denotes an MF75 formulation that includes MTP, such as from 100-400 µg MTP-PE per dose.

Submicron oil-in-water emulsions, methods of making the same and immunostimulating agents, such as muramyl peptides, for use in the compositions, are described in detail in International Publication No. WO90/14837 and US Patent Nos. 6,299,884 and 6,45 1,325, incorporated herein by reference in their entireties.

Complete Freund's adjuvant (CFA) and incomplete Freund's adjuvant (IFA) may also be used as adjuvants in the invention.

### C. Saponin Formulations

Saponin formulations, may also be used as adjuvants in the invention. Saponins are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs.

Saponin compositions have been purified using High Performance Thin Layer Chromatography (HP-LC) and Reversed Phase High Performance Liquid Chromatography (RP-HPLC). Specific purified fractions using these techniques have been identified, including QS7, QS17, QS18, QS21, QH-A, QH-B and QH-C. Preferably, the saponin is QS21. A method of production of QS21 is disclosed in US Patent No. 5,057,540. Saponin formulations may also comprise a sterol, such as cholesterol (see WO96/33739).

Combinations of saponins and cholesterols can be used to form unique particles called Immunostimulating Complexs (ISCOMs). ISCOMs typically also include a phospholipid such as phosphatidylethanolamine or phosphatidylcholine. Any known saponin can be used in ISCOMs. Preferably, the ISCOM includes one or more of Quil A, QHA and QHC. ISCOMs are further described in EP0109942, WO96/11711 and WO96/33739. Optionally, the ISCOMS may be devoid of additional detergent. See WO00/07621.

A review of the development of saponin based adjuvants can be found at Barr, et al., "ISCOMs and other saponin based adjuvants", Advanced Drug Delivery Reviews (1998) 32:247-271. See also Sjolander, et al., "Uptake and adjuvant activity of orally delivered saponin and ISCOM vaccines", Advanced Drug Delivery Reviews (1998) 32:321-338.

### D. Virosomes and Virus Like Particles (VLPs)

Virosomes and Virus Like Particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qβ-phage (such as coat proteins), GA-phage, fr-phage, AP205 phage, and Ty (such as retrotransposon Ty protein p1). VLPs are discussed further in WO03/024480, WO03/024481, and Niikura et al., "Chimeric Recombinant Hepatitis E Virus-Like Particles as an Oral Vaccine Vehicle Presenting Foreign Epitopes", Virology (2002) 293:273-280; Lenz et al., "Papillomarivurs-Like Particles Induce Acute Activation of Dendritic Cells", Journal of Immunology (2001) 5246-5355; Pinto, et al., "Cellular Immune Responses to Human Papillomavirus (HPV)-16 L1 Healthy Volunteers Immunized with Recombinant HPV-16 L1 Virus-Like Particles", Journal of Infectious Diseases (2003) 188:327-338; and Gerber et al., "Human Papillomavrisu Virus-Like Particles Are Efficient Oral Immunogens when Coadministered with Escherichia coli Heat-Labile Entertoxin Mutant R192G or CpG", Journal of Virology (2001) 75(10):4752-4760. Virosomes are discussed further in, for example, Gluck et al., "New Technology Platforms in the Development of Vaccines for the Future", Vaccine (2002) 20:B10 -B16.

Immunopotentiating reconstituted influenza virosomes (IRIV) are used as the subunit antigen delivery system in the intranasal trivalent INFLEXAL™ product {Mischler & Metcalfe (2002) *Vaccine* 20 Suppl 5:B17-23} and the INFLUVAC PLUS™ product.

### E. Bacterial or Microbial Derivatives

Adjuvants suitable for use in the invention include bacterial or microbial derivatives such as:
*1. Non-toxic derivatives of enterobacterial lipopolysaccharide (LPS)*
   Such derivatives include Monophosphoryl lipid A (MPL) and 3-O-deacylated MPL (3dMPL). 3dMPL is a mixture of 3 De-O-acylated monophosphoryl lipid A with 4, 5 or 6 acylated chains. A preferred "small particle" form of 3 De-O-acylated monophosphoryl lipid A is disclosed in EP 0 689 454. Such "small particles" of 3dMPL are small enough to be sterile filtered through a 0.22 micron membrane (see EP 0 689 454). Other non-toxic LPS derivatives include monophosphoryl lipid A mimics, such as aminoalkyl glucosaminide phosphate derivatives *e.g.* RC-529. See Johnson et al. (1999) Bioorg Med Chem Lett 9:2273-2278.
*2. Lipid A Derivatives*
   Lipid A derivatives include derivatives of lipid A from *Escherichia coli* such as OM-174. OM-174 is described for example in Meraldi et al., "OM-174, a New Adjuvant with a Potential for Human Use, Induces a Protective Response with Administered with the Synthetic C-Terminal Fragment 242-310 from the circumsporozoite protein of Plasmodium berghei", Vaccine (2003) 21:2485-2491; and Pajak, et al., "The Adjuvant OM-174 induces both the migration and maturation of murine dendritic cells in vivo", Vaccine (2003) 21:836-842.
3. *Immunostimulatory oligonucleotides*
   Immunostimulatory oligonucleotides suitable for use as adjuvants in the invention include nucleotide sequences containing a CpG motif (a sequence containing an unmethylated cytosine followed by guanosine and linked by a phosphate bond). Bacterial double stranded RNA or oligonucleotides containing palindromic or poly(dG) sequences have also been shown to be immunostimulatory.
   The CpG's can include nucleotide modifications/analogs such as phosphorothioate modifications and can be double-stranded or single-stranded. Optionally, the guanosine may be replaced with an analog such as 2'-deoxy-7-deazaguanosine. See Kandimalla, et al., "Divergent synthetic nucleotide motif recognition pattern: design and development of potent immunomodulatory oligodeoxyribonucleotide agents with distinct cytokine induction profiles", Nucleic Acids Research (2003) 31(9): 2393-2400; WO02/26757 and WO99/62923 for examples of possible analog substitutions. The adjuvant effect of CpG oligonucleotides is further discussed in Krieg, "CpG motifs: the active ingredient in bacterial extracts?", Nature Medicine (2003) 9(7): 831-835; McCluskie, et al., "Parenteral and mucosal prime-boost immunization strategies in mice with hepatitis B surface antigen and CpG DNA", FEMS Immunology and Medical Microbiology (2002) 32:179-185; WO98/40100; US Patent No. 6,207,646; US Patent No. 6,239,116 and US Patent No. 6,429,199.
   The CpG sequence may be directed to TLR9, such as the motif GTCGTT or TTCGTT. See Kandimalla, et al., "Toll-like receptor 9: modulation of recognition and cytokine induction by novel synthetic CpG DNAs", Biochemical Society Transactions (2003) 31 (part 3): 654-658. The CpG sequence may be specific for inducing a Th1 immune response, such as a CpG-A ODN, or it may be more specific for inducing a B cell response, such a CpG-B ODN. CpG-A and CpG-B ODNs are discussed in Blackwell, et al., "CpG-A-Induced Monocyte IFN-gamma-Inducible Protein-10 Production is Regulated by Plasmacytoid Dendritic Cell Derived IFN-alpha", J. Immunol. (2003) 170(8):4061-4068; Krieg, "From A to Z on CpG", TRENDS in Immunology (2002) 23(2): 64-65 and WO01/95935. Preferably, the CpG is a CpG-A ODN.
   Preferably, the CpG oligonucleotide is constructed so that the 5' end is accessible for receptor recognition. Optionally, two CpG oligonucleotide sequences may be attached at their 3' ends to form "immunomers". See, for example, Kandimalla, et al., "Secondary structures in CpG oligonucleotides affect immunostimulatory activity", BBRC (2003) 306:948-953; Kandimalla, et al., "Toll-like receptor 9: modulation of recognition and cytokine induction by novel synthetic GpG DNAs", Biochemical Society Transactions (2003) 31(part 3):664-658; Bhagat et al., "CpG penta-and hexadeoxyribonucleotides as potent immunomodulatory agents" BBRC (2003) 300:853-861 and WO03/035836.
*4. ADP-ribosylating toxins and detoxified derivatives thereof.*
   Bacterial ADP-ribosylating toxins and detoxified derivatives thereof may be used as adjuvants in the invention. Preferably, the protein is derived from *E. coli* (i.e., E. coli heat labile enterotoxin "LT), cholera ("CT"), or pertussis ("PT"). The use of detoxified ADP-ribosylating toxins as mucosal adjuvants is described in WO95/17211 and as parenteral adjuvants in WO98/42375. Preferably, the adjuvant is a detoxified LT mutant such as LT-K63, LT-R72, and LTR192G. The use of ADP-ribosylating toxins and detoxified derivaties thereof, particularly LT-K63 and LT-R72, as adjuvants can be found in the following references, each of which is specifically incorporated by reference herein in their entirety: Beignon, et al., "The LTR72 Mutant of Heat-Labile Enterotoxin of Escherichia coli Enahnces the Ability of Peptide Antigens to Elicit CD4+ T Cells and Secrete Gamma Interferon after Coapplication onto Bare Skin", Infection and Immunity (2002) 70(6):3012-3019; Pizza, et al., "Mucosal vaccines: non toxic derivatives of LT and CT as mucosal adjuvants", Vaccine (2001) 19:2534-2541; Pizza, et al., "LTK63 and LTR72, two mucosal adjuvants ready for clinical trials" Int. J. Med. Microbiol (2000) 290(4-5):455-461; Scharton-Kersten et al., "Transcutaneous Immunization with Bacterial ADP-Ribosylating Exotoxins, Subunits and Unrelated Adjuvants", Infection and Immunity (2000) 68(9):5306-5313; Ryan et al., "Mutants of Escherichia coli Heat-Labile Toxin Act as Effective Mucosal Adjuvants for Nasal Delivery of an Acellular Pertussis Vaccine: Differential Effects of the Nontoxic AB Complex and Enzyme Activity on Th1 and Th2 Cells" Infection and Immunity (1999) 67(12):6270-6280; Partidos et al., "Heat-labile enterotoxin of Escherichia coli and its site-directed mutant LTK63 enhance the proliferative and cytotoxic T-cell responses to intranasally co-immunized synthetic peptides", Immunol. Lett. (1999) 67(3):209-216; Peppoloni et al., "Mutants of the Escherichia coli heat-labile enterotoxin as safe and strong adjuvants for intranasal delivery of vaccines", Vaccines (2003) 2(2):285-293; and Pine et al., (2002) "Intranasal immunization with influenza vaccine and a detoxified mutant of heat labile enterotoxin from Escherichia coli (LTK63)" J. Control Release (2002) 85(1-3):263-270. Numerical reference for amino acid substitutions is preferably based on the alignments of the A and B subunits of ADP-ribosylating toxins set forth in Domenighini et al., Mol. Microbiol (1995) 15(6):1165-1167, specifically incorporated herein by reference in its entirety.

### F. Bioadhesives and Mucoadhesives

Bioadhesives and mucoadhesives may also be used as adjuvants in the invention. Suitable bioadhesives include esterified hyaluronic acid microspheres (Singh et al. (2001) J. Cont. Rele. 70:267-276) or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention. E.g. WO99/27960.

### G. Microparticles

Microparticles may also be used as adjuvants in the invention. Microparticles (i.e. a particle of ~100nm to ~150µm in diameter, more preferably ~200nm to ~30µm in diameter, and most preferably ~500nm to -10pm in diameter) formed from materials that are biodegradable and non-toxic (e.g. a poly(α-hydroxy acid), a polyhydroxybutyric acid, a polyorthoester, a polyanhydride, a polycaprolactone, etc.), with poly(lactide-co-glycolide) are preferred, optionally treated to have a negatively-charged surface (e.g. with SDS) or a positively-charged surface (e.g. with a cationic detergent, such as CTAB).

### H. Liposomes

Examples of liposome formulations suitable for use as adjuvants are described in US Patent No. 6,090,406, US Patent No. 5,916,588, and EP 0 626 169.

### I. Polyoxyethylene ether and Polyoxyethylene Ester Formulations

Adjuvants suitable for use in the invention include polyoxyethylene ethers and polyoxyethylene esters. WO99/52549. Such formulations further include polyoxyethylene sorbitan ester surfactants in combination with an octoxynol (WO01/21207) as well as polyoxyethylene alkyl ethers or ester surfactants in combination with at least one additional non-ionic surfactant such as an octoxynol (WO01/21152).

Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### J. Polyphosphazene (PCPP)

PCPP formulations are described, for example, in Andrianov et al., "Preparation of hydrogel microspheres by coacervation of aqueous polyphophazene solutions", Biomaterials (1998) 19(1-3):109-115 and Payne et al., "Protein Release from Polyphosphazene Matrices", Adv. Drug. Delivery Review (1998) 31(3):185-196.

### K. Muramyl peptides

Examples of muramyl peptides suitable for use as adjuvants in the invention include N-acetyl-muramyl-L-threonyl-D-isoglutamine (thr-MDP), N-acetyl-normuramyl-1-alanyl-d-isoglutamine (nor-MDP), and N-acetylmuramyl-1-alanyl-d-isoglutaminyl-1-alanine-2-(1'-2'-dipalmitoyl-sn-glycero-3-hydroxyphosphoryloxy)-ethylamine MTP-PE).

### L. Imidazoquinolone Compounds

Examples of imidazoquinolone compounds suitable for use adjuvants in the invention include Imiquamod and its homologues, described further in Stanley, "Imiquimod and the imidazoquinolones: mechanism of action and therapeutic potential" Clin Exp Dermatol (2002) 27(7):571-577 and Jones, "Resiquimod 3M", Curr Opin Investig Drugs (2003) 4(2):214-218.

The invention may also comprise combinations of aspects of one or more of the adjuvants identified above. For example, the following adjuvant compositions may be used in the invention:
(1) a saponin and an oil-in-water emulsion (WO99/11241);
(2) a saponin (e.g.., QS21) + a non-toxic LPS derivative (e.g. 3dMPL) (see WO94/00153);
(3) a saponin (e.g.., QS21) + a non-toxic LPS derivative (e.g. 3dMPL) + a cholesterol;
(4) a saponin (e.g. QS21) + 3dMPL + IL-12 (optionally + a sterol) (WO98/57659);
(5) combinations of 3dMPL with, for example, QS21 and/or oil-in-water emulsions (See European patent applications 0835318, 0735898 and 0761231);
(6) SAF, containing 10% Squalane, 0.4% Tween 80, 5% pluronic-block polymer L121, and thr-MDP, either microfluidized into a submicron emulsion or vortexed to generate a larger particle size emulsion.
(7) Ribi™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and
(8) one or more mineral salts (such as an aluminum salt) + a non-toxic derivative of LPS (such as 3dPML).

### M. Human Immunomodulators

Human immunomodulators suitable for use as adjuvants in the invention include cytokines, such as interleukins (e.g. IL-1, IL-2, IL-4, IL-5, IL-6, IL-7, IL-12, etc.), interferons (e.g. interferon-γ), macrophage colony stimulating factor, and tumor necrosis factor.

Aluminum salts and MF59 are preferred adjuvants for use with injectable influenza vaccines. Bacterial toxins and bioadhesives are preferred adjuvants for use with mucosally-delivered vaccines, such as nasal vaccines.

The following example illustrates generation of neutralizing antibodies by administration of low doses of rabies virus vaccine in a Thai Red Cross Indtradermal immunization regimen.

### EXAMPLES

### Generation of neutralizing antibodies by administration of low dose of PCECV in TRC ID regimen.

This study was conducted to evaluate the amount of antigen required to elicit a satisfactory immune response. As the administration of Human Rabies Immunoglobulin (HRIG) has been reported to reduce the immune response to vaccine in Post-Exposure Treatment, HRIG was given concomitantly in this clinical trial to evaluate the worst case scenario.

The study which was performed at the Infectious Deseases Department at the University Hospital of Hradec Kralove (Czech Republic) from October 2003 to January 2004 is a prospective, open-label, randomized, controlled, single-center, clinical trial. All tests conducted in this trial were performed in accordance with the GCP-ICH guidelines after approval of the Ethics Committee and the national authorities.

PCECV was administered to healthy human subjects (n=165) using a simulated post-exposure TRC ID regimen in combination with human rabies immunoglobulin. Briefly, PCECV (potency 5.1 IU/ml) was administered in ID doses of 0.1 mL, either undiluted or in dilutions from 1:2 up to 1:16 (i.e. up to approximately 0.032 IU/dose). Specifically, the PCECV corresponded to 0.51 IU/ID 0.1 mL dose (undiluted), 0.25/ID 0.1 mL dose (diluted 1:2; this potency corresponds to the minimal potency of 2.5 IU per IM dose recommended by the WHO), 0.125 IU/ID 0.1 mL dose (diluted 1:4), 0.062/ID 0.1 mL dose (diluted 1:8) and 0.031 IU/ID 0.1 mL dose (diluted 1:16).

The administration was performed in accordance with the 2-site intradermal regimen as depicted in figure 1 using seperate syringes and needles for each dose. On day 0, human rabies immunoglobulin was administered intragluteally in a concentration of 20 IU/mL (for example, Berirab®, obtainable from Chiron Behring, Marburg, Germany). No serious adverse drug reactions were observed during the regimen.

Blood was withdrawn from subjects on Days 0, 7, 14, 30, 90 and 104 and rabies virus neutralizing antibodies were determined using the rapid fluorescent focus inhibition test (RFFIT).

The results revealed an excellent and fast seroprotection rate. The percentage of subjects having an adequate titer of neutralizing antibodies (≥ 0.5 IU/mL) were calculated. On Day 14, as expected, 100% of the subjects receiving undiluted vaccine had titers above 0.5 IU/ml. Also all subjects receiving 1:2 diluted vaccine achieved protective titers. In the 1:4 and 1:8 groups all but one subjects had titers ≥ 0.5 IU/ml, whereas in the 1:16 group again 100% seroprotection was achieved. Exactly the same seroprotection rates were found on Day 30 with 100% seroprotection in the 1:16 group.

## Claims

1. Composition for use in the post-exposure vaccination of a human subject who has been exposed to rabies, said composition comprising a rabies virus vaccine, wherein the concentration of rabies virus antigen in the vaccine is less than 2.5 IU/ml, and wherein the composition is for intradermal administration in doses of 0.1 ml according to the Thai Red Cross regimen (2-2-2-0-1-1), wherein the numerical abbreviations refer to the numbers of doses administered on days 0, 3, 7, 14, 28 and 90 of the vaccination regimen, or the modified Thai Red Cross regimen (2-2-2-0-2), with the 90 day booster being emitted.

2. Composition for use according to claim 1, wherein the concentration of rabies virus antigen in the vaccine is less than one half of 2.5 IU/ml.

3. Composition for use according to claim 2, wherein the concentration of rabies virus antigen in the vaccine is less than one fourth of 2.5 IU/ml.

4. Composition for use according to claim 3, wherein the concentration of rabies virus antigen in the vaccine is less than one eighth of 2.5 IU/ml.

5. Composition for use according to claim 1, comprising a rabies virus vaccine, wherein the concentration of rabies virus antigen in the vaccine is between 2.4 IU/ml and 0.25 IU/ml.

6. Composition for use according to claim 5, wherein the concentration of rabies virus antigen in the vaccine is between 2.0 IU/ml and 1.0 IU/ml.

7. Composition for use according to claim 6, wherein the concentration of rabies virus antigen in the vaccine is between 1.75 IU/ml and 1.25 IU/ml.

8. Composition for use according to claim 7, wherein the concentration of rabies virus antigen in the vaccine is 1.5 IU/ml.

9. Composition for use according to claims 1-8, wherein the rabies virus vaccine is selected from the group consisting of purified chicken embryo cell vaccine (PCECV), purified vero cell vaccine (PVRV), and human diploid cell vaccine (HDCV).

10. Composition for use according to claims 1-9, wherein post-exposure vaccination is accompanied by the administration of rabies immune globulin.

## Patentansprüche

1. Zusammensetzung zur Verwendung bei der postexpositionellen Impfung eines humanen Individuums, das der Tollwut ausgesetzt war, wobei die Zusammensetzung einen Tollwutvirus-Impfstoff enthält, und wobei die Konzentration des Tollwutvirus-Antigens in dem Impfstoff weniger als 2,5 IU/ml beträgt, und wobei die Zusammensetzung für die intradermale Verabreichung in Dosen von 0,1 ml nach dem Thai Red Cross-Verabreichungsschema (2-2-2-0-1-1), wobei sich die numerischen Abkürzungen auf die Anzahl der zu verabreichenden Dosen an den Tagen 0, 3, 7, 14, 28 und 90 des Impfschemas beziehen, oder nach dem modifizierten Thai Red Cross-Verabreichungsschema (2-2-2-0-2), bei dem die Auffrischungsimpfung am Tag 90 ausgelassen wird, ist.

2. Zusammensetzung zur Verwendung nach Anspruch 1, wobei die Konzentration von Tollwutvirus-Antigen in dem Impfstoff weniger als die Hälfte von 2,5 IU/ml beträgt.

3. Zusammensetzung zur Verwendung nach Anspruch 2, wobei die Konzentration von Tollwutvirus-Antigen in dem Impfstoff weniger als ein Viertel von 2,5 IU/ml beträgt.

4. Zusammensetzung zur Verwendung nach Anspruch 3, wobei die Konzentration von Tollwutvirus-Antigen in dem Impfstoff weniger als ein Achtel von 2,5 IU/ml beträgt.

5. Zusammensetzung zur Verwendung nach Anspruch 1, die ein Tollwutvirus-Impfstoff umfasst, wobei die Konzentration des Tollwutvirus-Antigens in dem Impfstoff zwischen 2,4 IU/ml und 0,25 IU/ml beträgt.

6. Zusammensetzung zur Verwendung nach Anspruch 5, wobei die Konzentration des Tollwutvirus-Antigens in dem Impfstoff zwischen 2,0 IU/ml und 1,0 IU/ml beträgt.

7. Zusammensetzung zur Verwendung nach Anspruch 6, wobei die Konzentration des Tollwutvirus-Antigens in dem Impfstoff zwischen 1,75 IU/ml und 1,25 IU/ml beträgt.

8. Zusammensetzung zur Verwendung nach Anspruch 7, wobei die Konzentration des Tollwutvirus-Antigens in dem Impfstoff 1,5 IU/ml beträgt.

9. Zusammensetzung zur Verwendung nach den Ansprüchen 1-8, wobei der Tollwutvirus-Impfstoff ausgewählt ist aus der Gruppe bestehend aus einem Impfstoff aus gereinigten Hühnerembryozellen (purified chicken embryo cell vaccine, PCECV), einem Impfstoff aus gereinigten Verozellen (purified vero cell vaccine, PVRV) und einem Impfstoff aus humanen diploiden Zellen (human diploid cell vaccine, HDCV)

10. Zusammensetzung zur Verwendung gemäß den Ansprüchen 1-9, wobei die postexpositionelle Impfung gemeinsam mit der Verabreichung von Tollwut-Immunglobulin erfolgt.

## Revendications

1. Composition destinée à être utilisée dans la vaccination post-exposition d'un sujet humain qui a été exposé à la rage, ladite composition comprenant un vaccin antirabique, dans laquelle la concentration d'antigène viral rabique dans le vaccin est inférieure à 2,5 UI/ml, et dans laquelle la composition est destinée à être administrée par voie intradermique à des doses de 0,1 ml conformément au schéma d'administration de la Croix-Rouge thaïlandaise (2-2-2-0-1-1), dans lequel les abréviations numériques font référence au nombre de doses administrées aux jours 0, 3, 7, 14, 28 et 90 du schéma de vaccination ou selon le schéma d'administration modifié de la Croix-Rouge thaïlandaise (2-2-2-0-2) en omettant le rappel à 90 jours.

2. Composition destinée à être utilisée selon la revendication 1, dans laquelle la concentration d'antigène viral rabique dans le vaccin est inférieure à la moitié de 2,5 UI/ml.

3. Composition destinée à être utilisée selon la revendication 2, dans laquelle la concentration d'antigène viral rabique dans le vaccin est inférieure à un quart de 2,5 UI/ml.

4. Composition destinée à être utilisée selon la revendication 3, dans laquelle la concentration d'antigène viral rabique dans le vaccin est inférieure à un huitième de 2,5 UI/ml.

5. Composition destinée à être utilisée selon la revendication 1, comprenant un vaccin antirabique, dans laquelle la concentration d'antigène viral rabique dans le vaccin se situe entre 2,4 UI/ml et 0,25 UI/ml.

6. Composition destinée à être utilisée selon la revendication 5, dans laquelle la concentration d'antigène viral rabique dans le vaccin se situe entre 2,0 UI/ml et 1,0 UI/ml.

7. Composition destinée à être utilisée selon la revendication 6, dans laquelle la concentration d'antigène viral rabique dans le vaccin se situe entre 1,75 UI/ml et 1,25 UI/ml.

8. Composition destinée à être utilisée selon la revendication 7, dans laquelle la concentration d'antigène viral rabique dans le vaccin est de 1,5 UI/ml.

9. Composition destinée à être utilisée selon les revendications 1 à 8, dans laquelle le vaccin antirabique est choisi dans le groupe constitué par du vaccin purifié préparé sur cellules d'embryon de poulet (PCECV), du vaccin purifié préparé sur cellules Vero (PVRV) et du vaccin préparé sur cellules diploïdes humaines (HDCV).

10. Composition destinée à être utilisée selon les revendications 1 à 9, dans laquelle la vaccination post-exposition s'accompagne d'une administration d'immunoglobuline rabique.
